# EUROPEAN PATENT SPECIFICATION

(11) **EP 2 648 612 B1**
(45) Date of publication and mention of the grant of the patent: **10.12.2014**
(21) Application number: 11760812.5
(22) Date of filing: 09.09.2011
(51) Int. Cl.: A61B 5/055, A61B 6/00, A61B 6/04, A61B 19/00

(54) **BREAST IMMOBILISATION APPARATUS AND METHOD FOR USE IN BREAST IMAGING TO ALLOW MEDICAL INTERVENTION**
BRUSTIMMOBILISIERUNGSVORRICHTUNG UND VERFAHREN ZUR VERWENDUNG IN DER BRUSTBILDGEBUNG FÜR MEDIZINISCHE EINGRIFFE
APPAREIL D'IMMOBILISATION MAMMAIRE ET SON PROCÉDÉ D'UTILISATION EN IMAGERIE MAMMAIRE POUR FACILITER UNE INTERVENTION MÉDICALE

(30) Priority: 10.09.2010 GB 201015165
(43) Date of publication of application: 16.10.2013
(73) Proprietor: Specialty Magnetics Limited, Northolt, Middlesex UB5 5DY (GB)
(72) Inventor: AKGUN, Ali, Northolt MX UB5 5DY (GB)
(74) Representative: Prock, Thomas
(86) International application number: PCT/GB2011/001333
(87) International publication number: WO 2012/032308

(56) References cited:
- WO-A1-93/17620
- WO-A1-97/18751
- WO-A1-2010/092565
- WO-A2-95/08293
- WO-A2-2004/006755
- WO-A2-2005/117551
- DE-A1- 19 610 802
- US-A- 5 971 998
- US-A1- 2009 259 122

## Description

The present invention relates to a breast immobilisation apparatus for use in breast imaging to allow medical intervention.

In the process of imaging breasts it is usually necessary to gently immobilise the breasts to ensure that the image quality is not interfered with due to movement of the breasts. Movements of the patient during imaging can in turn result in the movement of the breast. If the breast is not immobilised during the performance of interventional procedures under real-time image guidance, interventional probes (e.g. biopsy needle) can also cause movement of the breast during their insertion into the breast. Prior art breast immobilisation techniques include squashing or sandwiching the breast between the two compression-plates or compression-grids whilst the patient in a standing or prone position. This bi-lateral method of immobilisation is not desirable since it potentially causes flattening and merging of the breast lesions. It is usually also necessary to position the breasts in accordance with the special requirements of the imaging modality (e.g. x-ray, magnetic resonance, ultrasound). It is desirable that the imaging modalities used should - in addition to the breast - also be able to image the chest-wall and axillary-tail anatomies of the patients.

Magnetic Resonance Imaging (MRI) is considered to be a method of imaging which has high sensitivity in terms of detecting smaller lesion in the breast and also high specificity in terms of characterising the nature of the lesions. Typically the patient must be inserted in a cylindrical MRI magnet, although more recently ultra-short magnets also having a large bore have become available (see WO01/70109). Such MRI systems have the advantages that the patient feels less claustrophobic and the patient is more accessible to medical staff during diagnostic imaging and more importantly during the performance of medical interventions which require access to the breasts during imaging. The performance of interventional procedures under real-time MRI guidance is generally only possible in the MRI systems employing ultra-short and large-bore magnets due to the physical constraints of the conventional, axially-long cylindrical magnet based MRI systems.

WO96/08199 discloses a device for guiding a medical intervention tool. A patient lies prone on a body support platform with their breasts pendulantly protruding through holes in the platform. One at a time the breast can be immobilised for imaging using a contractible basket which comprises two basket members which are urged together. The basket members have holes in them to allow medical intervention tools to be inserted there through. This device is for use with axially-long, narrow-bore conventional MRI systems and hence it is designed to enable an image to be taken of the immobilised breast. After initial imaging for the localisation of the lesion(s), the patient is then removed from the bore of the MRI machine to allow medical intervention via the holes in the basket members. Since the targeting of the lesion(s) by the interventional tool such as a probe has to happen when the patient is outside the MRI system, the conventional MRI system do not allow interactive medical intervention under image guidance.

The basket members in WO96/08199 immobilise the breast but also deform it since the two basket members are urged together. This deformation of the breast distorts the true shape of the morphology of the breast and can lead to lesions and the like being merged together by the force of the distorting compression. This distortion can also impact deleteriously on the medical intervention process.
Document WO9718751 discloses an apparatus according to the preamble of claim 1.

The present invention as defined by claim 1 aims to provide an improved breast immobilisation apparatus for use in breast diagnostic imaging and in the performance of (real time and non-real time) image-guided medical interventional procedures.

Thus, this aspect of the present invention overcomes the deficiencies in the prior art and provides for the immobilisation and support of the breast in its more natural shape in a breast cup which is designed to better match the natural shape of a breast. The cup shape can be ellipsoidal, dome shaped or hemispherical. Different shapes and sizes can be provided for patients. The aperture arrangement ensures that medical staff can access the breast through the breast cup to carry out medical intervention techniques during imaging if possible or after imaging if necessary.

In one embodiment the breast cup includes a flange running around an upper edge of the rigid structure, the upper edge defines an opening through which the breast is received, the breast cup is adapted to be inserted through the hole in the body support platform, and the flange is adapted to engage with an upper surface of the body support platform to hold the breast cup extending below the body support platform. This allows for simple exchange of the breast cup to use a breast cup to suit the size and shape of a patient's breast.

In one embodiment the body support platform has a flexible upper surface to mould to the shape of the prone patient. This ensures improved patient comfort and more importantly it ensures that the majority of the breast of the patient fits into the breast cup

In one embodiment the body support platform has a second hole for receiving a second breast of the patient, the apparatus including a second said breast cup arranged below the second hole. Thus the present invention enables single or double breast immobilisation and imaging.

In one embodiment the breast cup is disposable.

In one embodiment the body support platform comprises an upper surface having the hole therethrough and a base for placement on a conventional imaging body support platform, the base being connected to the upper surface to space the upper surface from the base below the hole to provide room for the breast cup received in the hole.

One aspect of the invention provides a breast imaging arrangement comprising the breast immobilisation apparatus, a medical intervention tool for medical intervention of the breast, and a tool support arrangement for supporting the medical intervention tool and enabling movement of the medical intervention tool to contact any required point on the surface of the breast.

This aspect of the present invention makes medical intervention simpler since the medical intervention tool is supported on the apparatus.

In one embodiment the breast cup includes MRI reference markers detectable during imaging, the apparatus including a processing arrangement for receiving a selection of a location within the breast to be targeted by the medical intervention tool and data on the detected reference markers, and a tool support driving mechanism for driving the tool support arrangement, wherein the processing arrangement is adapted to calculate drive signals and to control the tool support driving mechanism to automatically locate the medical intervention tool at the location to be targeted. This embodiment enabled automatic 'robotic' positioning of the medical intervention tool at the correct location for the medical intervention within the breast.

In one embodiment the breast imaging arrangement, includes an MRI magnet arrangement having a large bore and ultra-short axial length therefore allowing the placement of the patient and the apparatus within the bore of the magnet and also providing access to the breast by the medical staff, the breast immobilisation apparatus, the tool support arrangement and the medical intervention tool are arranged in the bore for medical intervention during imaging, and the MRI magnet arrangement includes a detecting antenna arranged around the breast cup for detection of MRI signals from the breast.

Another aspect of the dislosure provides a breast immobilisation method comprising for use in breast imaging to allow medical intervention, the method comprising supporting the body of a patient in a prone position, pendulantly projecting a breast of the patient through a hole in the body support platform; and receiving the breast of the patient in a breast cup, uniformly compressing the breast to immobilise it without substantially deforming the breast, and providing an aperture arrangement to allow access to a substantial portion of the surface of the breast for medical intervention, wherein uniformly compressing the breast comprises using a rigid structure and a resilient urging arrangement on an inner side of the rigid structure for urging into engagement with the breast for compression of the breast, and the aperture arrangement comprises an array of apertures in the rigid structure or gaps between parts of the rigid structure. Another aspect of the disclosure provides a breast imaging method comprising immobilising the breast of a patient using the breast immobilisation method, inserting the patient in an MRI magnet arrangement having a large bore and ultra-short axial length therefore allowing the placement of the patient and the apparatus within the bore of the magnet and also providing access to the breast by medical staff, arranging a detecting antenna around the breast cup for detection of MRI signals from the breast, and using a medical intervention tool to access the breast for medical intervention through the breast cup during imaging.

Embodiments of the present invention will now be described with reference to the accompanying drawings, in which:
Figure 1 is a diagram of a rigid part of the breast cup according to one embodiment of the present invention;
Figure 2 is a diagram of the rigid part of the breast cup showing the insertion of the pneumatic compressible member according to the first embodiment of the present invention;
Figure 3 is a diagram of the complete breast cup according to the first embodiment of the present invention;
Figure 4 is a diagram showing different sized breast cups according to the first embodiment of the present invention;
Figure 5 is a diagram showing the patient support platform with a breast cup support arrangement beneath according to the first embodiment of the present invention;
Figure 6 is a diagram showing the detecting antennas on the breast cup support arrangement according to the first embodiment of the present invention;
Figure 7 is a diagram showing the insertion of two breast cups in the holes in the patient support platform according to the first embodiment of the present invention;
Figure 8 is a diagram illustrating the positioning of the breast cups in the breast cup support arrangement according to the first embodiment of the present invention;
Figure 9 is a diagram illustrating the process for inflating the pneumatic compressible members in the breast cups according to the first embodiment of the present invention;
Figure 10 is a diagram illustrating the pneumatic compressible members in an inflated state immobilising the breasts in the breast cups according to the first embodiment of the present invention;
Figure 11 is a schematic cross section through a breast in the breast cup before inflation of the pneumatic compressible members according to the first embodiment of the present invention;
Figure 12 is a schematic cross section through a breast in the breast cup after inflation of the pneumatic compressible members according to the first embodiment of the present invention;
Figures 13 and 14 are diagrams illustrating the medical intervention toll support mechanism for use in imaging a single breast according to a second embodiment of the present invention;
Figure 15 is a diagram illustrating the breast cup support arrangement according to the second embodiment of the present invention;
Figure 16 is a diagram illustrating the inflation of the pneumatic compressible member according to the second embodiment of the present invention;
Figure 17 is a diagram illustrating the breast immobilised in the breast cup and the medical intervention tool in operation in accordance with the second embodiment of the present invention;
Figure 18 is a diagram illustrating the breast cup support arrangement with the medical intervention tool support in accordance with the second embodiment of the present invention;
Figure 19 to 24 are diagrams illustrating a third embodiment of the present invention comprising an apparatus for retro fitment to a conventional patient support platform;
Figure 25 is a diagram illustrating an alternative invention;
Figure 26 is a diagram illustrating an alternative invention;
Figure 27a and 27b is a diagram illustrating a fourth embodiment of the present invention;
Figure 28 is a diagram illustrating a fifth embodiment of the present invention; and
Figures 29 to 31 are diagrams illustrating different antenna configurations according to embodiments of the invention.

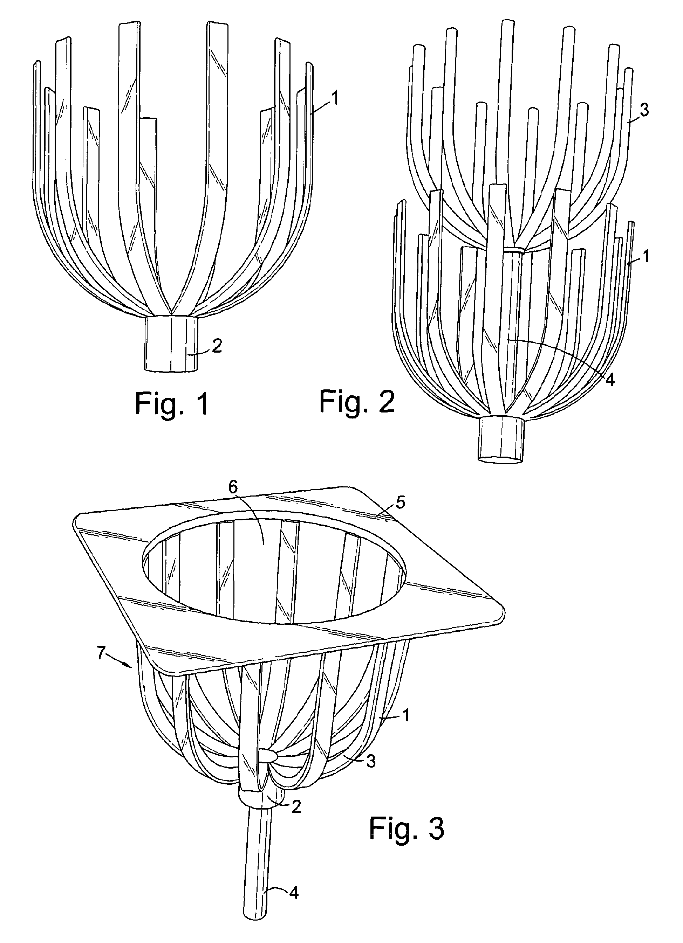

A first embodiment of the present invention will now be described with reference to figures 1 to 12. This embodiment enables the immobilisation of both breast of a patient at the same time to enable imaging of both breasts simultaneously in for example an MRI scanner.

Figure 1 illustrates a rigid structure 1 forming part of a breast cup. The rigid structure 1 comprises a plurality of finger-like projections radiating from a common point from which the finger-like projections are supported by a support sleeve 2. The finger-like projections radiate to form an outer surface of the breast cup. They form a generally dome shape extending from a cylindrical shape in this embodiment. The support sleeve 2 has an aperture to receive a resilient urging arrangement 3 as illustrated in figure 2. The resilient urging arrangement 3 comprises a plurality of finger-like projections matching the finger-like projections of the rigid structure 1. The finger-like projections of the resilient urging arrangement 3 are hollow and sealed at their top ends. At their bottom ends where they meet at a common point, they intercommunicate with each other and with a hollow tube 4. This provides a pneumatic or hydraulic structure which can be expanded (inflated or pumped up) via the tube 4. The tube 4 passes through the support sleeve 2 so that the resilient urging arrangement 3 is supported by the rigid structure 1. The resilient urging arrangement 3 can be fixed to the rigid structure 1 e.g. by adhesion or any other suitable fixing arrangement.

Figure 3 illustrates the complete structure of a breast cup in which a flange 5 is attached to upper ends of the rigid structure 1. The flange 5 is made of a rigid or slightly / semi flexible material and its purpose is to engage with an upper surface of a patient support platform around the periphery of an aperture in the surface of the patient support platform to enable the rigid structure 1 with attached resilient urging arrangement 3 to hang through aperture. The flange 5 has a breast aperture 6 therein corresponding to the dimensions of the cylindrical open end of the rigid structure 1.

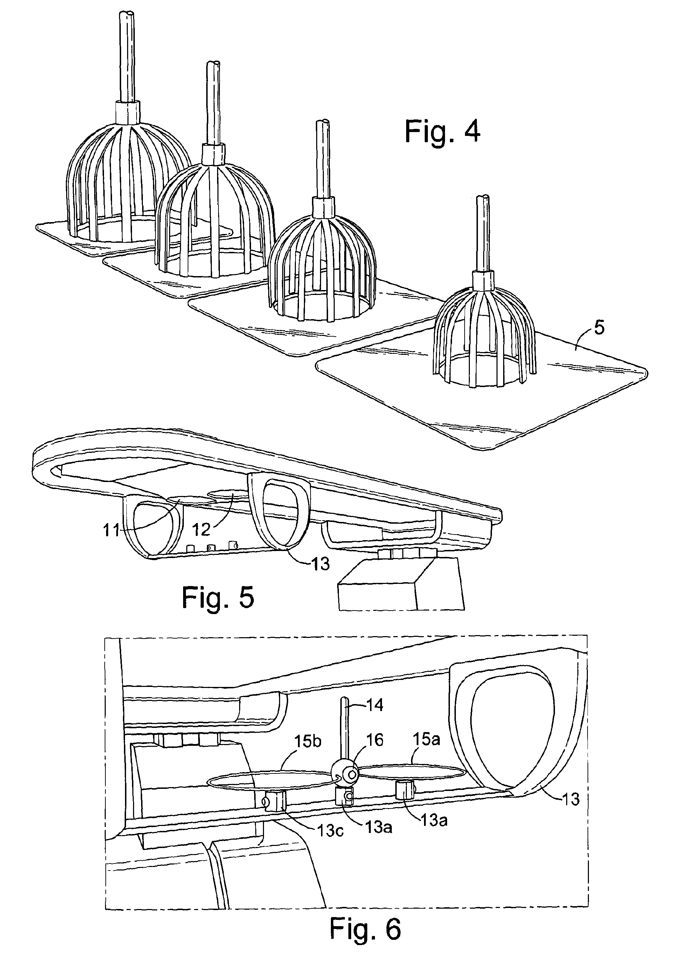

As can be seen in figure 4, different sized breast cups can be provided to accommodate the different breast sizes of the patients. The outer dimensions of the flange 5 remain the same for each of the different sized breast cups to engage with the periphery of the aperture in the patient support platform. Only the breast aperture 6 varies in size for the flanges 5 to match the variation in size of the rigid structure 1. The breast cup can be disposable. This ensures health and hygiene requirements are met. No other parts of the apparatus need be disposable or cleaned after every use since they do not come into contact with the patient.

Figure 5 illustrates a patient support platform 10 having apertures 11 and 12 for allowing the breasts of a patient to pendulantly project there through. Beneath the body support platform 10 and connected to a lower surface thereof is a support arrangement 13. The support arrangement 13 is arranged to support the breast cups, detection antennae, and a tool support arrangement as will be described in more detail herein after. The support arrangement 13 includes three spaced support connectors 13a, 13b and 13c. As can be seen in figure 6, a support rod 14 is supported in the middle support connector 13b and two antennae 15a and 15b are supported on the support rod 14 using a slidable and lockable sleeve 16. This enables the antennae to be raised or lowered relative to the support arrangement 13. The antennae 15a and 15b are supported to be coaxial with the outer two support connectors 13a and 13c.

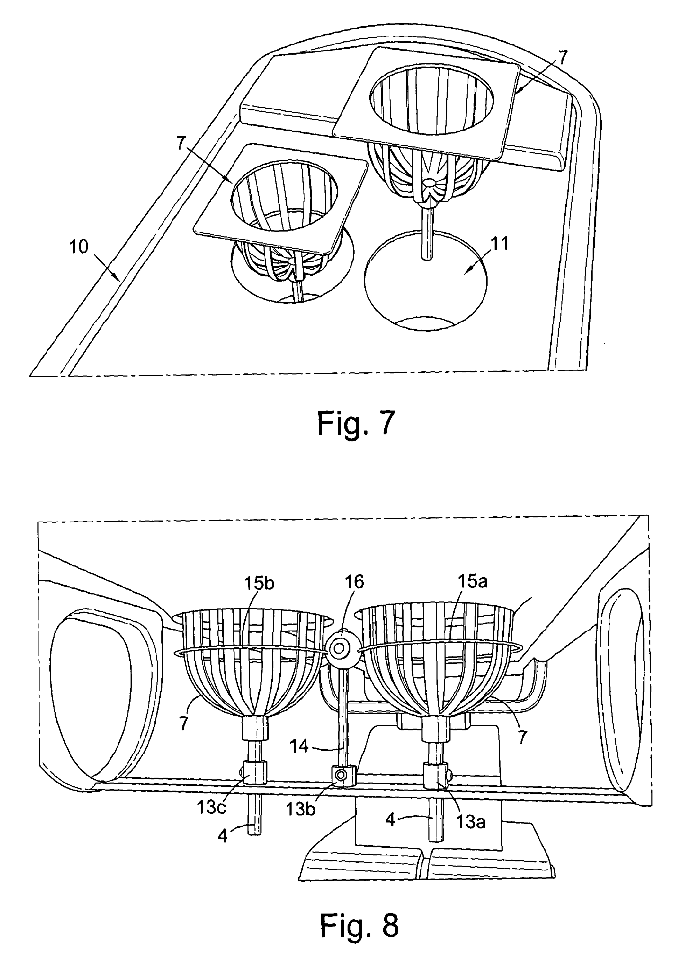

Figure 7 illustrates the insertion of two breast cups 7 into the apertures 11 and 12 in the patient support platform 10. Each breast cup 7 is inserted so that the tube 4 insert through a respective support connector 13a or 13c and the flange 5 lies flush with the upper surface of the patient support platform 10. The surface of the patient support platform 10 can be made of a flexible, semi-rigid or resilient material so that it conforms to the contours of the patient. It can for example be made of a sheet of resilient material such as rubber or a strong fabric material. This promotes better engagement of the breasts and ensures that the maximum breast material protrudes into the breast cups 7.

Figure 8 illustrates the breast cups 7 in position. Once they are in position the antennae 15a and 15b are raised up the rod 14 to surround the breast cups 7 to enable good detection of MRI signals from the breasts during MRI imaging in an MRI scanner.

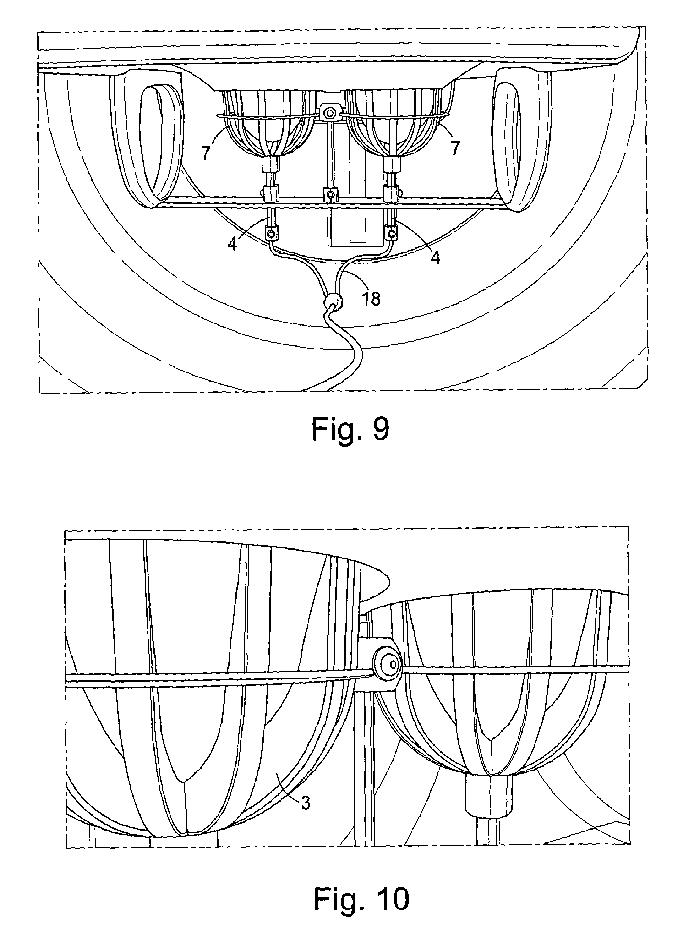

Figure 9 is a view under the patient support platform 10 with a patient lying prone with her breasts protruding into the breast cups 7. A pneumatic or hydraulic connection 18 is then made to the tubes 4 for expansion (inflation, pumping up etc) of the resilient urging arrangement 3. Figure 10 illustrates the resilient urging arrangement 3 of each breast cup 7 in an urging (inflated or pumped up) state so that an inner surface of the resilient urging arrangement 3 is in compressible contact with the surface of the patient's breasts.

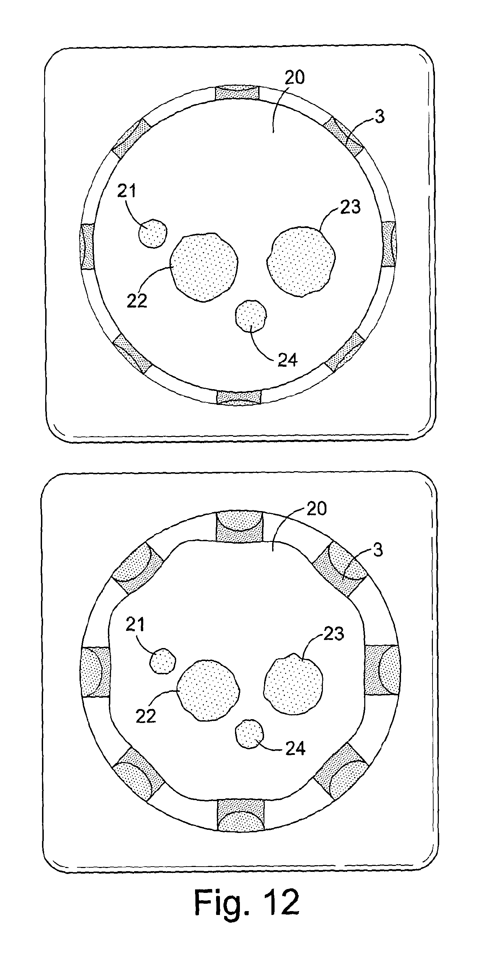

Figures 11 and 12 illustrate the effect of this embodiment of the invention on the breast of the patient. These figures schematically illustrate a section through a breast 20 of a patient in which lesions 21, 22, 23 and 24 are present. In figure 11 the breast is located in the breast cup 7 but the resilient urging arrangement is not in an urging state. Figure 12 illustrates the resilient urging arrangement 3 in an urging state in which radial compression of the breast takes place. This does not result in significant distortion of the breast and most importantly it does not result in significant distortion of the lesions 21, 22, 23 and 24. This omnidirectional and substantially uniform force applied to the breast 20 ensures that the breast is immobilised but kept in a natural shape, thereby keeping the anatomy of the breast in a natural shape.

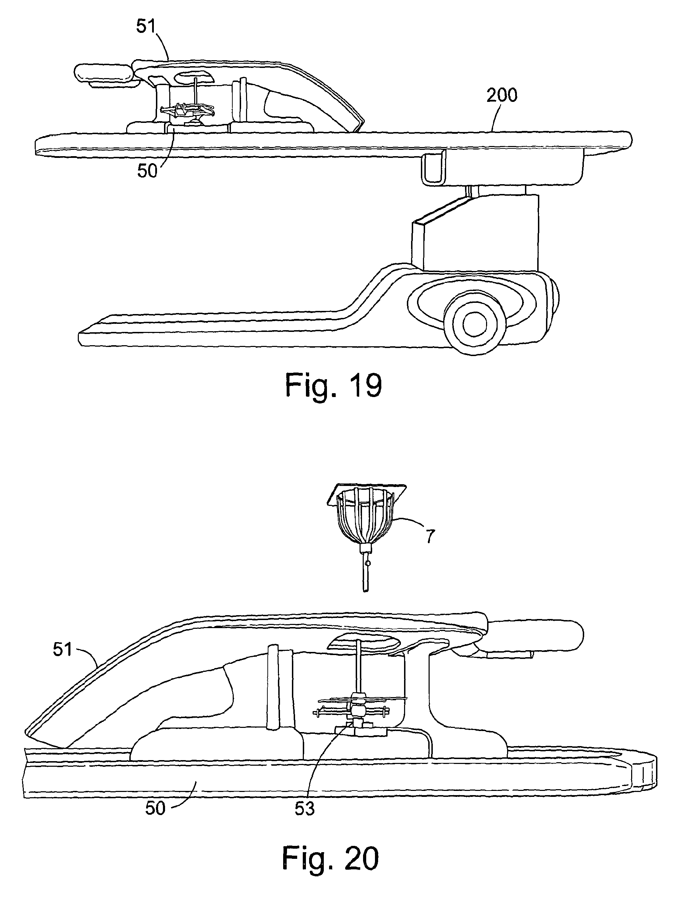

In this first embodiment both breasts can be imaged simultaneously. The support arrangement 13 can also be arranged to support a medical intervention support for supporting a medical intervention tool, in a similar arrangement depicted in Figures 19 to 24. However, for simplification, a structure for providing medical intervention tool support will be described in more detail with reference to a configuration for imaging a single breast.

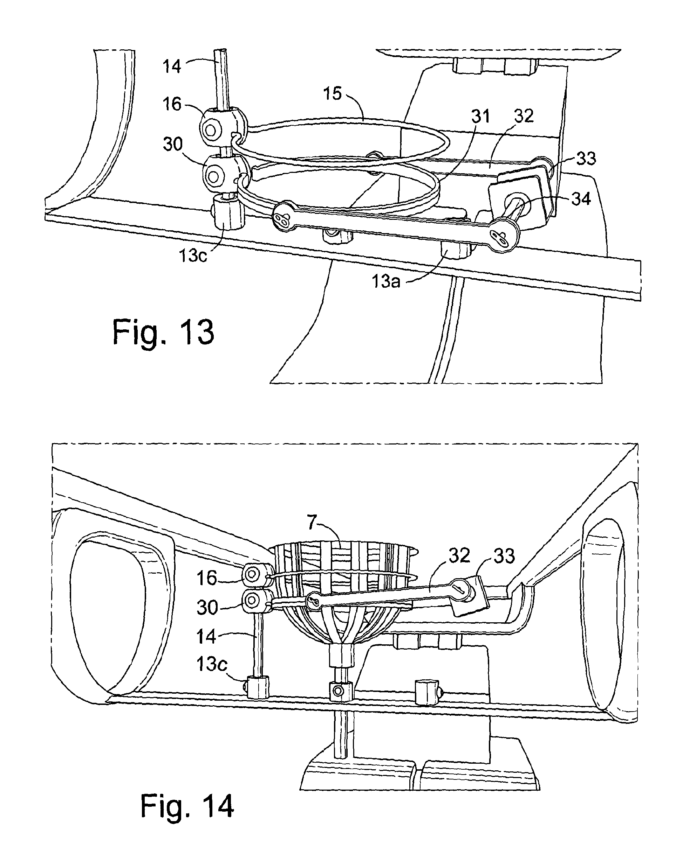

Figures 13 to 18 illustrate a second embodiment of the invention for imaging a single breast and providing for medical intervention. This embodiment uses the same breast cup structure 7 as for the first embodiment and the same support structure 13. Also the same slidable and lockable sleeve 16 with a single antenna 15 is used carried by the same rod 14. However, in this embodiment, the rod 14 carrying the antenna 15 is positioned in one of the outer support connectors 13c.

A medical intervention tool support structure is provided which comprises a slidable and lockable sleeve 30 on the rod 14 carrying a gimbal ring 31 carrying two arms 32 such that the arms 32 can rotate about the axis of the gimbal ring 31. The arms 32 are connected at a first end to the gimbal ring 31 by pivot joints so that the arms can rotate about an axis perpendicular to the axis of the gimbal ring 31. The arms 32 are connected at a second end to a rod 34 which carries a tool support block 33 which is rotatable about the rod 34 and slideable along the rod 34. The tool support block 33 can support any necessary medical intervention tool (not shown in figure 13). The tool support block 33 can be changed or modified to suit any medical intervention tool.

The structure of the medical intervention tool support structure enables a medical intervention tool to be positioned at any angle and in any direction to enable to the medical intervention tool to engage any part of the surface of the breast through gaps in the breast cup.

Figure 14 illustrates the arrangement of this embodiment after insertion of the breast cup 7 into the aperture in the patient support platform. The antenna 15 has been raised into its detecting position surrounding the breast cup 7. The medical intervention tool support structure has been raised also.

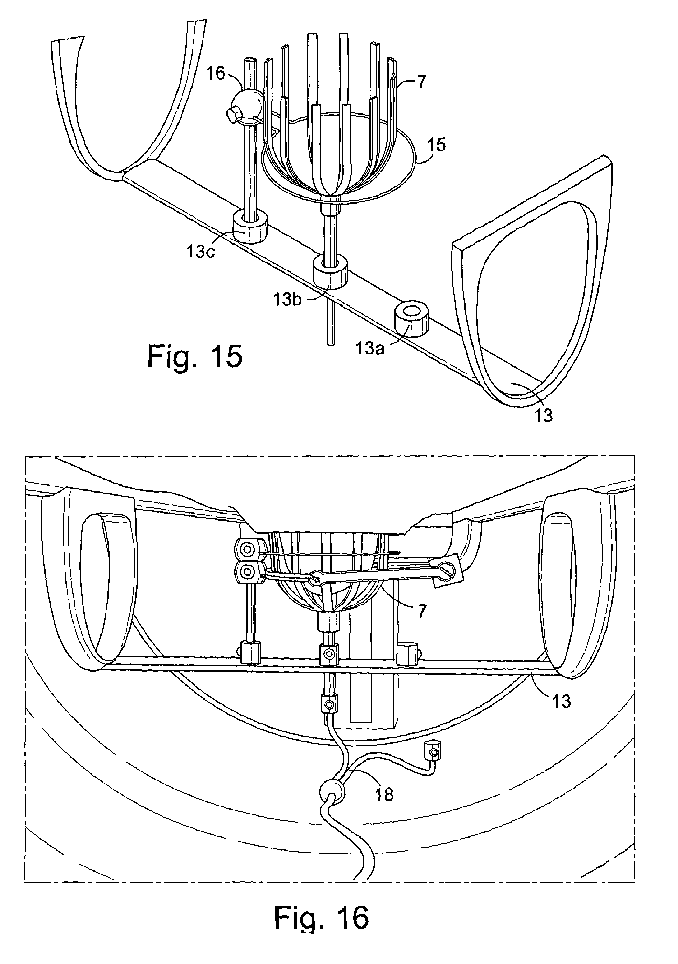

Figure 15 schematically illustrates the support structure 13 detached from the patient support platform 10 with the breast cup 7 (minus the flange 5) held in the support connector 13b.

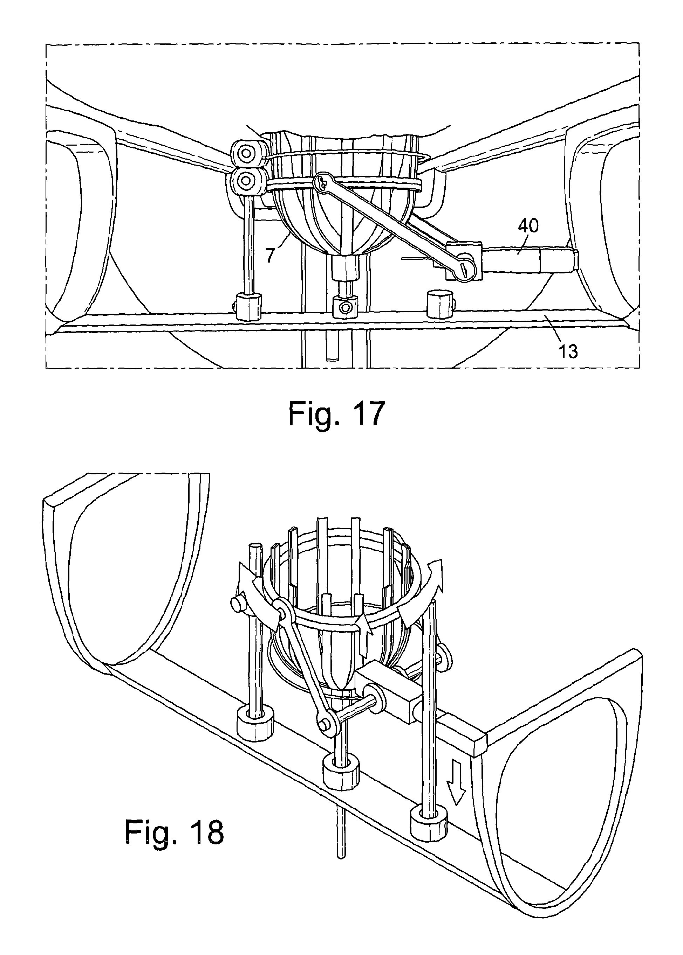

Figure 16 is a view under the patient support platform 10 with a patient lying prone with her breast protruding into the breast cup 7. A pneumatic or hydraulic connection 18 is then made to the tubes 4 for expansion (inflation, pumping up etc) of the resilient urging arrangement 3. Figure 17 illustrates the resilient urging arrangement 3 of the breast cup 7 in an urging (inflated or pumped up) state so that an inner surface of the resilient urging arrangement 3 is in compressible contact with the surface of the patient's breast. Also a medical intervention tool 40 is mounted on the medical intervention tool support arrangement. Figure 18 schematically illustrates the support structure 13 detached from the patient support platform 10 with the breast cup 7 (minus the flange 5) held in the support connector 13b.

Although in this embodiment a specific medical intervention tool support arrangement is disclosed, an alternative arrangement can comprise a robotic arm capable of moving in any direction around the breast cup.

Figures 19 to 24 illustrate a third embodiment of the present invention in which the apparatus can be added to a stand-alone, standard patient support platform that does not have one or two apertures to receive the patient's breasts.

This arrangement comprises a base 50 which is placed on the patient support platform 200. Supported by the base 50 and spaced there from is a body support surface 51 having two apertures therein to receive the patient's breasts. Below the body support surface 51 and connected to a lower surface thereof is a support arrangement 53. The support arrangement 53 is arranged to support the breast cups 7 and detection antennae 55a and 55b. The support arrangement 53 includes three spaced support connectors 53a, 53b and 53c. A support rod 54 is supported in the middle support connector 53b and two antennae 55a and 55b are supported on the support rod 54 using a slidable and lockable sleeve 56. This enables the antennae 55a and 55b to be raised or lowered relative to the support arrangement 53. The antennae 55a and 55b are supported to be coaxial with the outer two support connectors 53a and 53c.

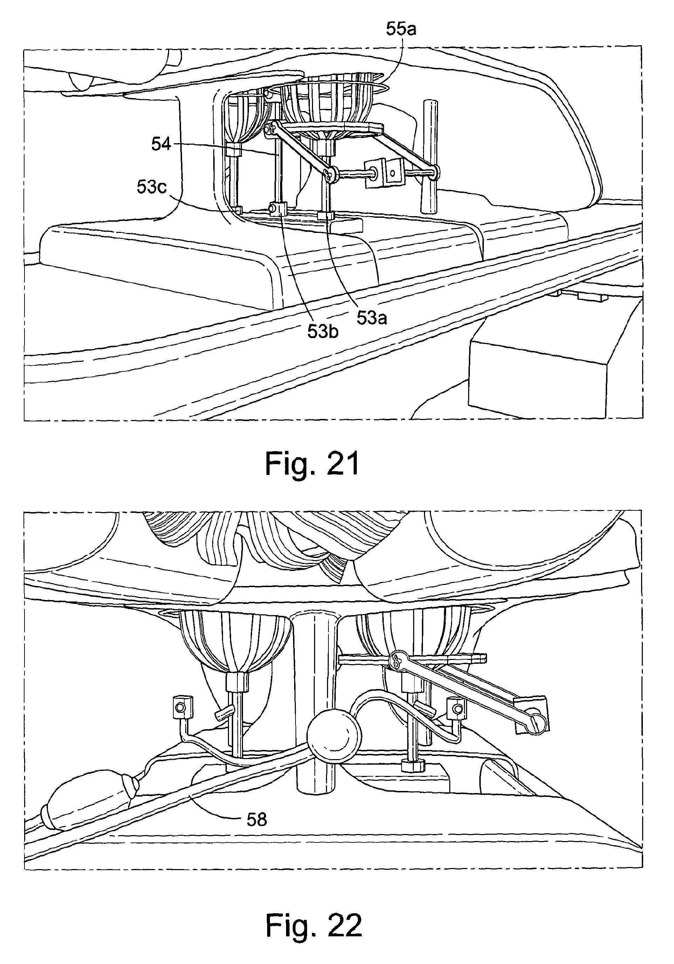

Figure 22 illustrates the connection of a pneumatic or hydraulic connection 58 for activation of the urging of the resilient urging arrangement of the breast cups 7 in a manner as described with reference to the first embodiment.

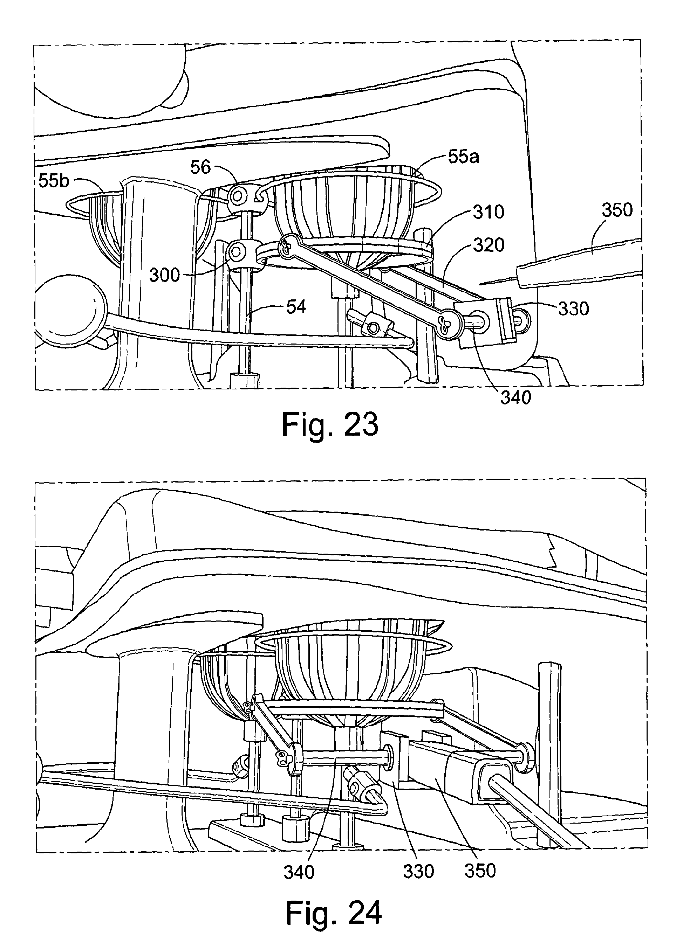

A medical intervention tool support structure is provided which comprises a slidable and lockable sleeve 300 on the rod 54 carrying a gimbal ring 310 carrying two arms 320 such that the arms 320 can rotate about the axis of the gimbal ring 310. The arms 320 are connected at a first end to the gimbal ring 310 by pivot joints so that the arms can rotate about an axis perpendicular to the axis of the gimbal ring 310. The arms 320 are connected at a second end to a rod 340 which carries a tool support block 330 which is rotatable about the rod 340 and slideable along the rod 340. The tool support block 330 can support any necessary medical intervention tool 350 shown approaching the tool support block 330 in figure 23. The tool support block 330 can be changed or modified to suit any medical intervention tool.

The structure of the medical intervention tool support structure enables a medical intervention tool to be positioned at any angle and in any direction to enable to the medical intervention tool to engage any part of the surface of the breast through gaps in the breast cup.

Thus this third embodiment enables the retro fitting of the apparatus to a conventional patient support platform for insertion into the bore of an MRI scanner.

In this embodiment the medical intervention tool support arrangement can be incorporated as described with reference to the first embodiment.

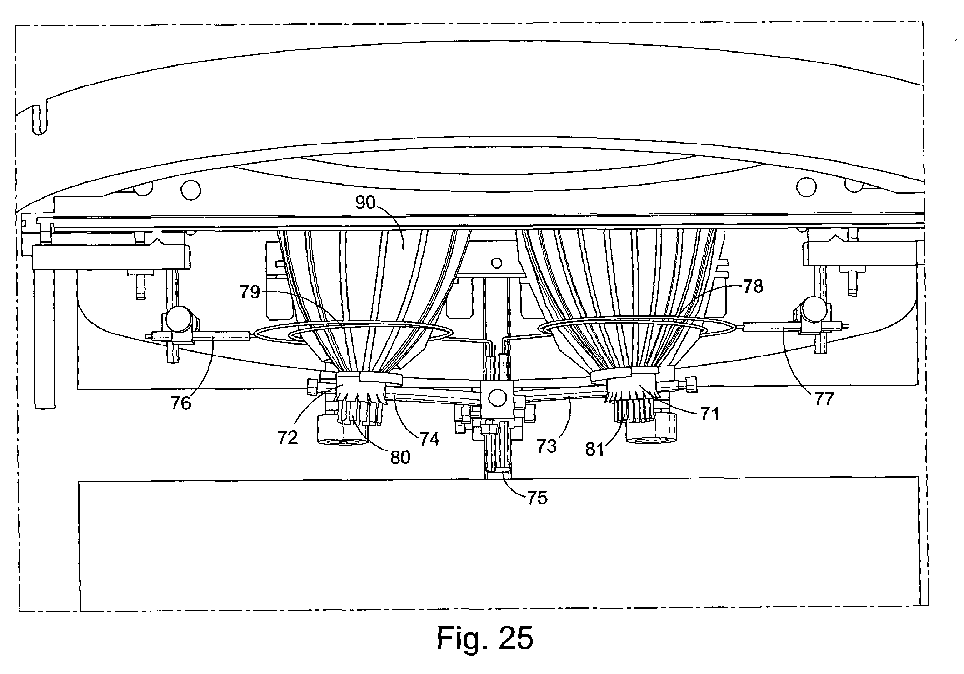

Figure 25 illustrates an alternative invention. In this invention a different configuration for the breast cups is used. The diagram is of a view from under the patient support platform. Two breast cups 70 are illustrated hanging from the underside of the patient support platform. The breast cups 70 comprise resilient tubes extending from the patient support platform downwards. The resilient tubes are made of resilient sheet material with vertical ribs 90 to provide enhanced rigidity in the vertical direction. The lower end of the tube may be of narrower diameter than the upper end. At the lower end of the tube an urging arrangement is provided comprising a compressing ring 71 and 72. Each compressing ring 71 and 72 is supported by arms 73 and 74 connected to a locking ring on a support rod 75. The compressing rings can be moved upwards to slide over the outer face of the resilient tubes to compress the ends so as to apply a compressing force on the breasts of the patient. Surplus resilient material 80 and 81 is drawn down through the compression rings 71 and 72 during the compression operation. The resilient tubes can be provided with apertures therein to allow for access to the breasts for medical intervention. Support arms 76 and 77 are provided to support antennae 78 and 79 which are arranged surrounding the breast cups 70.

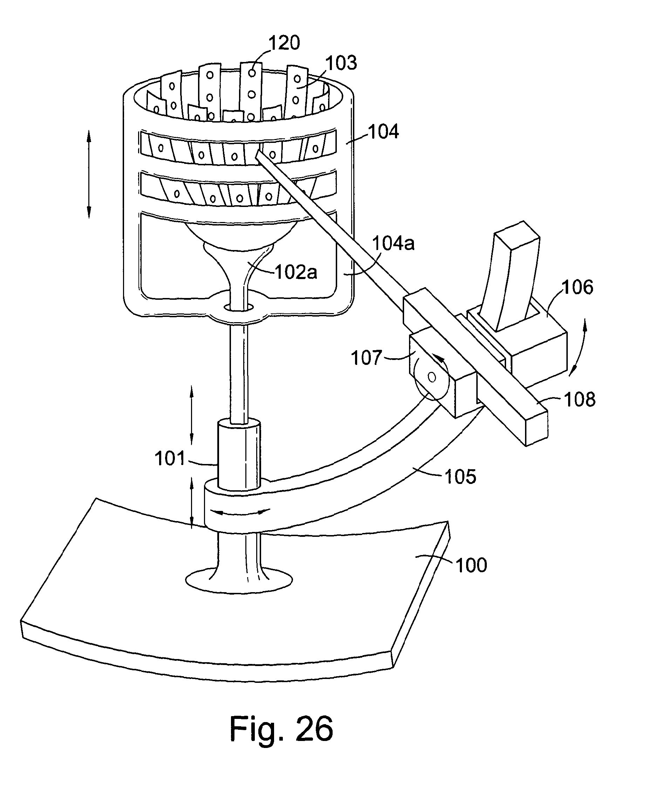

Figure 26 illustrates an alternative invention in which a support structure 100 is arranged below a patient support platform. A fixed support 101 extends from the support structure and an extendible support rod 102 is inserted in the fixed support 101. At the end of the extendible support rod 102 is arranged a flared portion 102a which carries a breast cup 103 in the form of a plurality of finger-like projections extending radially and upwardly from the flared portion 102a. Each finger-like portion has apertures therein to provide access to the surface of the breast for medical intervention. An antenna arrangement 104 comprises a number of coils, which in this specific example is three (but it can be any appropriate number) which is arranged around the breast cup 103. The antenna arrangement is mounted on an antenna support 104a which is slideably mounted on the extendible support rod 102 which allows the height of the breast cup 103. The extendible support rod allows the height of the breast cup 103 to be adjusted.

In use, a patient's breast is inserted in the breast cup 103. The antennae 104 are then moved from a lower position to an upper position where they surround the breast cup 103. In doing so the antennae 104 engage with the outer surface of the breast cup and urge the finger-like portions to compress the patient's breast. Alternatively the finger-like portions are sprung in a radial and axial direction such that they are held open during insertion of the breast and allowed to spring back to compress the breast in a direction towards the centre of the breast.

A medical intervention tool support arrangement is provided by an arm 105 pivoted on the fixed support 101. The arm curves in an arcuate manner upwards an away from the fixed support 101. A bracket 106 is slideably attached to the arm 105 to allow it to slide up and down the arm in an arc. A support member 107 is pivotally mounted on the bracket 106 to allow the support member to pivot about an axis perpendicular to the arc of the arm 105. A medical intervention tool 108 is mounted in the support member 107 for engagement with the surface of the breast through the gaps between the finger-like projections of the breast cup 103 or through the apertures 120 in the finger-like projections.

Although not shown in previous embodiments, the apertures 120 provided in the invention of figure 25 may be provided in the finger like projections 1 to enable the medical intervention tool to pass through. Where an inflatable or pneumatic lining is provided for gripping the breast in such a configuration, holes can be provided through this also to allow access to the breast there through.

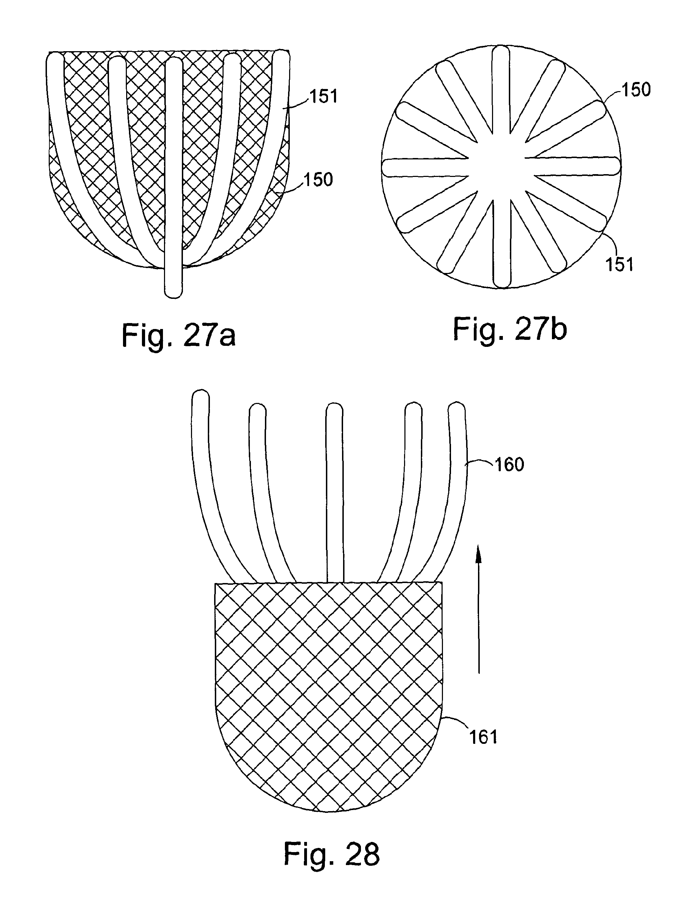

Figures 27a and 27b illustrate a breast cup of a fourth embodiment of the present invention. The breast cup of this embodiment comprises a rigid mesh 150 forming an outer part of the breast cup and finger-like projections 151 of hollow expandable material arranged on an inner surface extending from a common point at the base on the breast cup and extending radially and upwardly within the cup. The projections 151 interconnect at the common point 152 and can be inflated or expanded pneumatically or hydraulically from this point.

The breast cup of this embodiment is similar to the first embodiment except that the finger-like projections 1 are replaced with a rigid mesh. The breast cup of this embodiment can be used with the arrangement of the first and second embodiments.

Figure 28 illustrates a breast cup of a fifth embodiment of the present invention. In this embodiment resilient finger-like projections are connected at a common point and extend radially and upwardly. A rigid mesh cup 161 is arranged to be moveable vertically upwards over the finger-like projections to urge the projections radially inwardly to compress the breast of the patient.

In the fourth and fifth embodiments of the present invention, the use of the rigid mesh provides apertures through which medical intervention tools can gain access to the breast of the patient.

Where the apparatus of the embodiments is for use in an MRI scanner, the apparatus is formed from material that is MRI compatible.

The medical intervention tool using any of the embodiments can comprise a probe, a biopsy needle for obtaining a tissue sample from a suspicious lesion, or other trans-cannula procedures such as cryoablation, laser ablation, hyperthermia, medication delivery, percutaneous removal, draining. Further, the medical intervention tools can comprise surgical tolls for keyhole surgery.

The medical intervention tool support arrangement in any embodiment can be electromechanically driven under the control of a computer. The breast cup can contain imaging markers such as MRI markers that show up in the breast image. The position of these markers in the image relative to an area to which the medical intervention tool is to be guided can be determined by the computer. This information can be used to generate control signals to control the medical intervention tool support arrangement to move the medical intervention tool to an appropriate location in an appropriate orientation to approach a desired target area of a lesion in the breast to medical intervention.

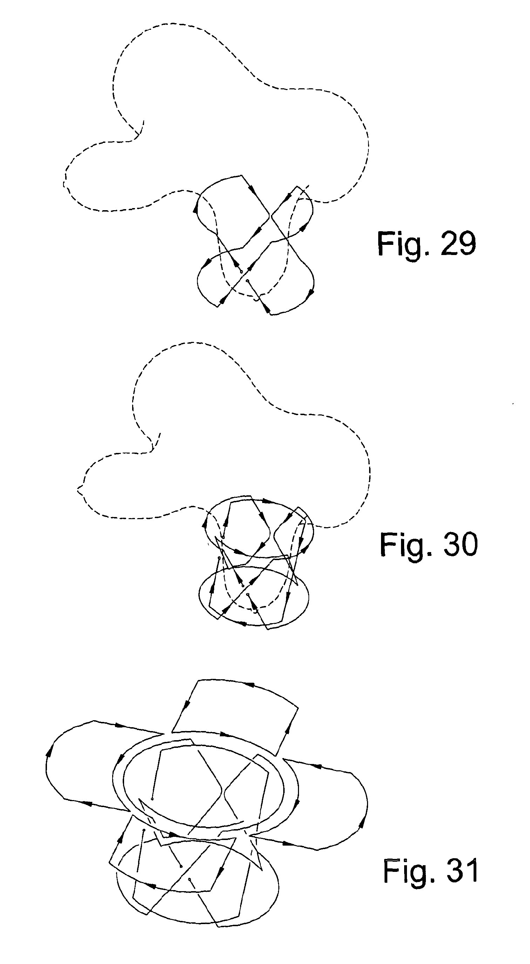

Although in all of these embodiments a specific detecting antenna arrangement is disclosed, as part of this invention, other alternative antenna designs, such as the ones illustrated in figures 29 to 30 can also be adopted and used.

## Claims

1. Breast immobilisation apparatus for use in breast imaging to allow medical intervention, the apparatus comprising
a body support platform (10) for supporting the body of a patient in a prone position, the body support platform (10) having a hole (11, 12) through which the breast of the patient is allowed to pendulantly project in use; and
a breast cup (7), comprising a rigid structure (1), said cup (7) arranged below the hole (11, 12) for receiving the breast of the patient and comprising means for compressing the breast to immobilise it, wherein the means for compressing the breast comprises said rigid structure (1) and resilient urging arrangement (3) for urging into engagement with the breast for compression of the breast, said resilient urging arrangement (3) arranged on an inner side of the rigid structure (1) and said rigid structure (1) comprising an aperture arrangement to allow access to a portion of the surface of the breast for medical intervention;
**CHARACTERISED**
**in that** the rigid structure (1) comprises a plurality of finger-like projections radiating from a common point (2);
the resilient urging arrangement (3) comprises a plurality of finger-like projections matching the finger-like projections of the rigid structure (1) and a hydraulic or pneumatic arrangement for expansion of the finger-like projections of the resilient urging arrangement (3) to urge against the surface of the breast; and
that the aperture arrangement is formed by gaps between the finger-like projections.

2. Breast immobilisation apparatus according to Claim 1, wherein the breast cup (7) includes a flange (5) running around an upper edge of the rigid structure (1), the upper edge defines an opening (6) through which the breast is received, the breast cup (7) is adapted to be inserted through the hole (11, 12) in the body support platform (10), and the flange (5) is adapted to engage with an upper surface of the body support platform (10) to hold the breast cup (7) extending below the body support platform (10).

3. Breast immobilisation apparatus according to claim 2, wherein the body support platform (10) has a flexible upper surface to mould to the shape of the prone patient.

4. Breast immobilisation apparatus according to claim 1 or 2, wherein the breast cup (7) comprises one of a plurality of different sized cups (7) selected for the breast size of the patient.

5. Breast immobilisation apparatus according to any preceding claim, wherein the body support platform (10) has a second hole (11, 12) for receiving a second breast of the patient, the apparatus including a second said breast cup (7) arranged below the second hole (11, 12).

6. Breast immobilisation apparatus according to any preceding claim, wherein the breast cup (7) is disposable.

7. Breast immobilisation apparatus according to any preceding claim, wherein the body support platform (10) comprises an upper surface having the hole (11, 12) there through and a base for placement on a conventional imaging body support platform (10), the base being connected to the upper surface to space the upper surface from the base below the hole (11, 12) to provide room for the breast cup (7) received in the hole (11, 12).

8. A breast imaging arrangement comprising the breast immobilisation apparatus according to any preceding claim, a medical intervention tool (40) for medical intervention of the breast, and a tool support (32, 33, 34) arrangement for supporting the medical intervention tool (40) and enabling movement of the medical intervention tool (40) to contact any required point on the surface of the breast.

9. A breast imaging arrangement according to claim 8, wherein the breast cup (7) includes MRI reference markers detectable during imaging, the apparatus including a processing arrangement for receiving a selection of a location within the breast to be targeted by the medical intervention tool (40) and data on the detected reference markers, and a tool support driving mechanism for driving the tool support arrangement (32, 33, 34), wherein the processing arrangement is adapted to calculate drive signals and to control the tool support driving mechanism to automatically locate the medical intervention tool (40) at the location to be targeted.

10. A breast imaging arrangement according to claim 8 or claim 9, including a MRI magnet arrangement having a bore, wherein an axial length of the magnet arrangement down the bore is less than a diameter of the bore, the breast immobilisation apparatus, the tool support arrangement (32, 33, 34) and the medical intervention tool (40) are arranged in the bore for medical intervention during imaging, and the MRI magnet arrangement includes a detecting antenna (15, 15a, 15b) arranged around the breast cup (7) for detection of MRI signals from the breast.

## Patentansprüche

1. Brustimmobilisierungsvorrichtung für eine Verwendung bei der Brustbildgebung, um einen medizinischen Eingriff zu gestatten, wobei die Vorrichtung aufweist:
eine Körperauflageplattform (10) für das Tragen des Körpers eines Patienten in einer Bauchlage, wobei die Körperauflageplattform (10) ein Loch (11, 12) aufweist, durch das die Brust des Patienten bei Benutzung hängend herausragen kann; und
ein Brustkörbchen (7), das eine starre Struktur (1) aufweist, wobei das Körbchen (7) unterhalb des Loches (11, 12) für das Aufnehmen der Brust des Patienten angeordnet ist, und wobei es ein Mittel für das Zusammendrücken der Brust aufweist, um sie unbeweglich zu machen, wobei das Mittel für das Zusammendrücken der Brust eine starre Struktur (1) und eine elastische Antriebsanordnung (3) für ein Ineingriffkommen mit der Brust für ein Zusammendrücken der Brust aufweist, wobei die elastische Antriebsanordnung (3) auf einer Innenseite der starren Struktur (1) angeordnet ist, und wobei die starre Struktur (1) eine Öffnungsanordnung aufweist, um einen Zugang zu einem Abschnitt der Oberfläche der Brust für einen medizinischen Eingriff zu gestatten;
**dadurch gekennzeichnet,**
**dass** die starre Struktur (1) eine Vielzahl von fingerartigen Vorsprüngen aufweist, die von einem gemeinsamen Punkt (2) aus strahlenförmig ausgehen;
wobei die elastische Antriebsanordnung (3) eine Vielzahl von fingerartigen Vorsprüngen, die zu den fingerartigen Vorsprüngen der starren Struktur (1) passen, und eine hydraulische oder pneumatische Anordnung für die Expansion der fingerartigen Vorsprünge der elastischen Antriebsanordnung (3) aufweist, um gegen die Oberfläche der Brust zu drücken; und dadurch,
**dass** die Öffnungsanordnung durch Lücken zwischen den fingerartigen Vorsprüngen gebildet wird.

2. Brustimmobilisierungsvorrichtung nach Anspruch 1, bei der das Brustkörbchen (7) einen Flansch (5) umfasst, der um einen oberen Rand der starren Struktur (1) verläuft, wobei der obere Rand eine Öffnung (6) definiert, durch die die Brust aufgenommen wird, wobei das Brustkörbchen (7) so ausgebildet ist, dass es durch das Loch (11, 12) in der Körperauflageplattform (10) eingesetzt wird, und wobei der Flansch (5) so ausgebildet ist, dass er mit einer oberen Fläche der Körperauflageplattform (10) in Eingriff kommt, um das Brustkörbchen (7) zu halten, das sich unterhalb der Körperauflageplattform (10) erstreckt.

3. Brustimmobilisierungsvorrichtung nach Anspruch 2, bei der die Körperauflageplattform (10) eine elastische obere Fläche aufweist, um sich nach der Form des in Bauchlage befindlichen Patienten zu gestalten.

4. Brustimmobilisierungsvorrichtung nach Anspruch 1 oder 2, bei der das Brustkörbchen (7) eines von einer Vielzahl von unterschiedlich bemessenen Körbchen (7) aufweist, die für die Brustgröße des Patienten ausgewählt werden.

5. Brustimmobilisierungsvorrichtung nach einem der vorhergehenden Ansprüche, bei der die Körperauflageplattform (10) ein zweites Loch (11, 12) für das Aufnehmen einer zweiten Brust des Patienten aufweist, wobei die Vorrichtung ein zweites Brustkörbchen (7) umfasst, das unterhalb des zweiten Loches (11, 12) angeordnet ist.

6. Brustimmobilisierungsvorrichtung nach einem der vorhergehenden Ansprüche, bei der das Brustkörbchen (7) wegwerfbar ist.

7. Brustimmobilisierungsvorrichtung nach einem der vorhergehenden Ansprüche, bei der die Körperauflageplattform (10) eine obere Fläche mit einem Loch (11, 12) dort hindurch und eine Basis für eine Anordnung auf einer konventionellen Körperauflageplattform (10) für die Bildgebung aufweist, wobei die Basis mit der oberen Fläche verbunden ist, um die obere Fläche von der Basis unterhalb des Loches (11, 12) mit Abstand anzuordnen, um einen Platz für das Brustkörbchen (7) bereitzustellen, das im Loch (11, 12) aufgenommen wird.

8. Brustbildgebungsanordnung, die aufweist: die Brustimmobilisierungsvorrichtung nach einem der vorhergehenden Ansprüche; ein medizinisches Eingriffswerkzeug (40) für einen medizinischen Eingriff an der Brust; und eine Werkzeughalterungsanordnung (32, 33, 34) für das Halten des medizinischen Eingriffswerkzeuges (40) und das Ermöglichen einer Bewegung des medizinischen Eingriffswerkzeuges (40), um eine erforderliche Stelle auf der Oberfläche der Brust zu kontaktieren.

9. Brustbildgebungsanordnung nach Anspruch 8, bei der das Brustkörbchen (7) MRI-Bezugsmarkierungen umfasst, die während der Bildgebung nachweisbar sind, wobei die Vorrichtung umfasst: eine Verarbeitungsanordnung für das Aufnehmen einer Auswahl einer Stelle innerhalb der Brust, die das Ziel des medizinischen Eingriffswerkzeuges (40) ist, und von Daten auf den nachgewiesenen Bezugsmarkierungen; und einen Werkzeughalterungsantriebsmechanismus für das Antreiben der Werkzeughalterungsanordnung (32, 33, 34), wobei die Verarbeitungsanordnung ausgebildet ist, um die Antriebssignale zu berechnen, und um den Werkzeughalterungsantriebsmechanismus zu steuern, um automatisch das medizinische Eingriffswerkzeug (40) an der Zielstelle anzuordnen.

10. Brustbildgebungsanordnung nach Anspruch 8 oder Anspruch 9, die eine MRI-Magnetanordnung mit einer Bohrung aufweist, wobei eine axiale Länge der Magnetanordnung entlang der Bohrung kleiner ist als ein Durchmesser der Bohrung, wobei die Brustimmobilisierungsvorrichtung, die Werkzeughalterungsanordnung (32, 33, 34) und das medizinische Eingriffswerkzeug (40) in der Bohrung für einen medizinischen Eingriff während der Bildgebung angeordnet sind, und wobei die MRI-Magnetanordnung eine Erfassungsantenne (15, 15a, 15b) umfasst, die um das Brustkörbchen (7) für ein Erfassen von MRI-Signalen von der Brust angeordnet ist.

## Revendications

1. Appareil d'immobilisation du sein destiné à être utilisé dans l'imagerie du sein pour permettre une intervention médicale, l'appareil comprenant :
une plate-forme de support du corps (10) pour supporter le corps d'un patient dans une position ventrale, la plate-forme de support du corps (10) comportant un trou (11, 12) à travers lequel le sein du patient peut déborder de manière retombante lors de l'emploi ; et
une coupelle pour le sein (7), comprenant une structure rigide (1), ladite coupelle (7) étant agencée au-dessous du trou (11, 12) pour recevoir le sein du patient, et comprenant un moyen pour comprimer le sein afin de l'immobiliser, le moyen destiné à comprimer le sein comprenant ladite structure rigide (1) et un dispositif de poussée élastique (3) en vue d'un engagement par poussée dans le sein afin de comprimer le sein, ledit assemblage de poussée élastique (3) étant agencé sur un côté interne de la structure rigide (1) et ladite structure rigide (1) comprenant un agencement d'ouverture pour donner accès à une partie de la surface du sein en vue d'une intervention médicale ;
**caractérisé**
**en ce que** la structure rigide (1) comprend plusieurs saillies en forme de doigt rayonnant à partir d'un point commun (2) ;
le dispositif de poussée élastique (3) comprend plusieurs saillies en forme de doigt complémentaires des saillies en forme de doigt de la structure rigide (1), et un dispositif hydraulique ou pneumatique en vue de l'expansion des saillies en forme de doigt du dispositif de poussée élastique (3), pour exercer une poussée contre la surface du sein ; et
**en ce que** l'agencement d'ouverture est formé par des espaces entre les saillies en forme de doigt.

2. Appareil d'immobilisation du sein selon la revendication 1, dans lequel la coupelle pour le sein (7) englobe une bride (5) s'étendant autour d'un bord supérieur de la structure rigide (1), le bord supérieur définissant une ouverture (6) à travers laquelle le sein est reçu, la coupelle pour le sein (7) étant adaptée pour être insérée à travers le trou (11, 12) dans la plate-forme de support du corps (10), et la bride (5) étant adaptée pour s'engager dans une surface supérieure de la plate-forme de support du corps (10), pour retenir la coupelle pour le sein (7) s'étendant au-dessous de la plate-forme de support du corps (10).

3. Appareil d'immobilisation du sein selon la revendication 2, dans lequel la plate-forme de support du corps (10) comporte une surface supérieure flexible en vue d'un moulage adapté à la forme du patient en position ventrale.

4. Appareil d'immobilisation du sein selon les revendications 1 ou 2, dans lequel la coupelle pour le sein (7) comprend plusieurs coupelles de taille différente (7), sélectionnées en fonction de la taille du sein du patient.

5. Appareil d'immobilisation du sein selon l'une quelconque des revendications précédentes, dans lequel la plate-forme de support du corps (10) comporte un deuxième trou (11, 12) pour recevoir un deuxième sein du patient, l'appareil englobant une deuxième coupelle pour le sein (7) agencée au-dessous du deuxième trou (11, 12).

6. Appareil d'immobilisation du sein selon l'une quelconque des revendications précédentes, dans lequel la coupelle pour le sein (7) est à usage unique.

7. Appareil d'immobilisation du sein selon l'une quelconque des revendications précédentes, dans lequel la plate-forme de support du corps (10) comprend une surface supérieure comportant le trou (11, 12) la traversant, et une base destinée à être placée sur une plate-forme de support d'un corps d'imagerie conventionnel (10), la base étant connectée à la surface supérieure pour espacer la surface supérieure de la base au-dessous du trou (11, 12) afin de ménager un espace pour la coupelle pour le sein (7) reçue dans le trou (11, 12).

8. Assemblage d'imagerie pour le sein, comprenant l'appareil d'immobilisation du sein selon l'une quelconque des revendications précédentes, un outil d'intervention médicale (40) en vue d'une intervention médicale sur le sein, et un assemblage de support de l'outil (32, 33, 34) pour supporter l'outil d'intervention médicale (40) et pour permettre le déplacement de l'outil d'intervention médicale (40) en vue d'un contact avec un quelconque point requis sur la surface du sein.

9. Assemblage d'imagerie du sein selon la revendication 8, dans lequel la coupelle pour le sein (7) englobe des marqueurs de référence IRM pouvant être détectés au cours de la formation d'images, l'appareil englobant un dispositif de traitement pour recevoir une sélection d'un emplacement dans le sein devant être ciblé par l'outil d'intervention médicale (40), et des données sur les marqueurs de référence détectés, et un mécanisme d'entraînement du support de l'outil pour entraîner l'assemblage de support de l'outil (32, 33, 34), le dispositif de traitement étant adapté pour calculer des signaux d'entraînement et pour contrôler le mécanisme d'entraînement du support de l'outil en vue de positionner automatiquement l'outil d'intervention médicale (40) au niveau de l'emplacement devant être ciblé.

10. Assemblage d'imagerie du sein selon les revendications 8 ou 9, englobant un assemblage d'aimant IRM comportant un alésage, dans lequel une longueur axiale de l'assemblage d'aimant le long de l'alésage est inférieure à un diamètre de l'alésage, l'appareil d'immobilisation du sein, l'assemblage de support de l'outil (32, 33, 34) et l'outil d'intervention médicale (40) étant agencés dans l'alésage en vue d'une intervention médicale au cours de la formation d'images, et l'assemblage d'aimant IRM englobant une antenne de détection (15, 15a, 15b) agencée autour de la coupelle pour le sein (7) pour détecter les signaux IRM du sein.
